# EUROPÄISCHE PATENTSCHRIFT

(11) **EP 0 436 885 B1**
(45) Veröffentlichungstag und Bekanntmachung des Hinweises auf die Patenterteilung: **14.06.1995**
(21) Anmeldenummer: 90124555.5
(22) Anmeldetag: 18.12.1990
(51) Int. Cl.: A61B 17/56, F16B 35/04

(54) **Schraube als Osteosynthesehilfsmittel**
Auxiliary osteosynthesis screw
Vis auxiliaire d'ostéosynthèse

(30) Priorität: 21.12.1989 DE 3942326
(43) Veröffentlichungstag der Anmeldung: 17.07.1991
(73) Patentinhaber: Härle, Anton, Prof. Dr., D-48161 Münster (DE)
(72) Erfinder: Härle, Anton, Prof. Dr., D-48161 Münster (DE)
(74) Vertreter: Habbel, Hans-Georg, Dipl.-Ing.

(56) Entgegenhaltungen:
- EP-A- 0 242 708
- WO-A-89/00028
- WO-A-89/04150
- DE-A- 251 246
- DE-A- 2 013 968
- DE-A- 3 027 138
- DE-U- 8 713 879
- FR-A- 2 642 958
- US-A- 3 488 779
- US-A- 3 695 259
- US-A- 3 997 138
- US-A- 4 836 196

## Beschreibung

Die Erfindung betrifft eine Schraube gemäß dem Oberbegriff des Hauptanspruches.

Knochenschrauben bzw. Stifte mit Knochengewinde-Anteil werden meist in Kombination mit Platten- und Stabsystemen angewandt, um Knochen bzw. Knochenteile in einer bestimmten Stellung und Ausrichtung zueinander zu fixieren.

Bei der herkömmlichen Osteosynthese an Röhrenknochen wird dabei eine mit Löchern versehene Platte mittels durch diese Löcher hindurchgreifenden Knochenschrauben am Knochen fixiert. Die Ruhigstellung der Knochen bzw. Knochenteile erfolgt durch Anpressen an die Platte mittels Schrauben, wobei die Schraubenköpfe auf der dem Knochen gegenüberliegenden Seite in den angeschrägten Schraubenlöchern ein Widerlager finden. Die stabile Verbindung der Knochen bzw. Knochenteile wird also über eine Anpressung von Schraubenköpfen an die Knochenplatte und über die sekundär damit hervorgerufene Anpressung der Knochenplatte an den Knochen selbst erreicht. Die im Knochen verankerten Knochenschrauben werden in ihrem Verlauf im Knochen selbst praktisch nur auf Zug beansprucht, während in diesem Bereich die Biegekräfte durch das Knochengewebe aufgefangen werden. Am Austritt der Schrauben aus dem Knochen treten dann wesentlich größere, mechanische Beanspruchungen auf, wobei insbesondere Biegebelastungen einwirken. Der Übergang des Knochengewindes der herkömmlichen Schrauben stellt daher eine Beanspruchungskonzentration dar, wobei durch die Einarbeitung einer in den Schraubenhals hineinragenden Imbusausnehmung noch eine zusätzliche Materialschwächung hervorgerufen wird.

In der klinischen Praxis sind daher immer wieder Schraubenbrüche zu beobachten, die in typischer Weise an dieser Prädilektionsstelle auftreten. Da der im Knochen verbleibende, abgebrochene Schraubenanteil praktisch mit der Knochenoberfläche abschließt und keine Angriffsfläche bietet, müssen zu seiner Entfernung mittels Hohlbohrer relativ große Knochenaufbohrungen vorgenommen werden, die die Knochen erheblich schwächen.

Bei Fixationsverfahren mit Knochenschraubenstiften, die durch eine seitliche Verbindung an Stabsystemen fixiert werden, sind derartige Brüche wegen der größeren Elastizität und der bei einer breitflächigen Kontaktaufnahme geringer anfallenden Kräftekonzentration etwas seltener. Doch kommt es bei dem größeren Hebelarm und der längeren Wegstrecke zwischen Knochenaustritts- und Fixationsstelle am Stabsystem zu Verbiegungen der Stifte und folglich zu einer mangelnden Ruhigstellung der Knochen bzw. Knochenanteile. Unerwünschte Stellungsänderungen und ausbleibende Knochenheilung sind die Folge.

Bei Verplattungsverfahren an der Wirbelsäule ist die Beanspruchung der Knochenschrauben an ihrem Übergang zum Schraubenkopf noch größer, da aufgrund der anatomischen Verhältnisse jeder Knochen nur durch eine Knochenschraube an der Knochenplatte fixiert werden kann. Ein weiteres Stabilitätsdefizit resultiert daraus, daß die Knochenplatten nicht breitflächig an Knochen-Kontaktflächen angepreßt werden können, um so einen Teil der Biegungsbelastung durch Zug-/Druck-Beanspruchung zu kompensieren. Da einerseits erhebliche Kräfte und Belastungen auftreten (nahezu das ganze Körpergewicht lastet auf einer fixierenden Schraube), andererseits die Anpressung zwischen Platte und Knochen und sekundär zwischen Platte und Schraube stark herbgesetzt ist, kann eine für die knöcherne Konsolidierung erforderliche Ruhigstellung oft nicht erreicht werden. Wegen der punktuellen Überlastung der Schrauben unter der vermehrten Biegebelastung lockern sie aus oder brechen viel häufiger als in der Osteosynthese an den Extremitäten. Die Auslockerung wiederum hat ihre Ursache darin, daß eine solide Verankerung nur im Kontaktbereich der Knochenschraube mit kortikalen (Knochenrinde) Strukturen gegeben ist. Während bei den Röhrenknochen der Extremitäten die Schraube immer in zwei Knochenrinden verankert wird, werden bei der Wirbelsäulenfixation die Schrauben durch die engen Knochenverbindungen zwischen dem vorn liegenden Wirbelkörper und dem hinten liegenden Wirbelbögen eingedreht. Diese Knochenbrücken, Bogenwurzeln (Pedikel) genannt, haben die Form einer Zwirnspule, wobei nur im mittleren, d.h. engen Abschnitt eine gute, direkte Kraftübertragung auf die zentral verlaufende und nur hier mit der Knochenrinde tangentialen Kontakt aufnehmende Knochenschraube erfolgen kann. Dorsal dieses Isthmuses ist eine wesentliche Kraftübertragung kaum mehr möglich, insbesondere liegt auch keine die Knochenschraube an ihrem Austritt abstützende Knochenrinde vor.

Eine stabile Fixation ist an der Wirbelsäule schon primär schwierig und nur dann möglich, wenn das Gewinde der Knochenschraube mit der Pedikel-Kortikalis im Isthmusbereich direkten Kontakt aufnimmt. Die Beanspruchungskonzentration, die natürlich von der Solidität der knöchernen Schraubenverankerung abhängig ist, tritt entweder am Schraubenkopf, meist aber schon einige Millimeter vom Schraubenkopf entfernt dort auf, wo die Schraube ihren Kontakt mit der Isthmus-Kortikalis verliert. Insbesondere bei winkelstabilen Verankerungen der Knochenschrauben an einer Knochenplatte sind diese Konzentrationen in hohem Maße geeignet, Schraubenbrüche im hinteren Pedikelbereich in rd. 10 % der klinischen Anwendungen nach sich zu ziehen, wobei die Brüche etwa 2 - 8 mm vor dem Austritt der Schraube aus dem Pedikel, d.h. in diesem auftreten. Wegen der direkten Nähe des Rückenmarks sind derartige Komplikationen besonders problematisch und schwer zu beherrschen.

Aus der EP-325 682 A1 ist eine Knochenschraube bekanntgeworden, bei welcher der Bereich unterhalb der Handhabe verstärkt ausgebildet ist, wobei sich aber der Außendurchmesser des Knochengewindebereiches nicht erweitert und die Verstärkung unterhalb der Handhabe nicht als Gewinde ausgebildet ist, so daß beim Einschrauben der Schraube hier ein besonders intensives Verdrängen des umgebenden Knochenbereiches erfolgen muß, ohne daß sich die Schraube in diesem Bereich verankert.

Der Erfindung liegt daher die Aufgabe zugrunde, eine Schraube als Osteosynthesehilfsmittel zu schaffen, die eine große Stabilität aufweist, sich besonders stabil verankert und leicht zu betätigen ist.

Diese der Erfindung zugrundeliegende Aufgabe wird durch die Lehre des Hauptanspruches gelöst.

Vorteilhafte Ausgestaltungen sind in den Unteransprüchen erläutert.

Ausführungsbeispiele der Erfindung werden nachfolgend anhand der Zeichnungen erläutert. Die Zeichnungen zeigen dabei in
- Fig. 1: eine Schraube, bei welcher der Knochengewindebereich sich zur Handhabe hin im oberen Bereich erweitert, in
- Fig. 2: eine Schraube, bei welcher sich an die Handhabe eine Verlängerung, die mit Maschinengewinde ausgerüstet ist, anschließt, in
- Fig. 3: eine am Übergang zwischen Maschinengewindebereich und Handhabe einzusetzende stabilisierende Hülse, in
- Fig. 4: eine mit dem Maschinengewindebereich der Schraube zusammenwirkende Mutter, in
- Fig. 5: eine abgeänderte Ausführungsform einer mit dem Maschinengewindebereich zusammenwirkenden Mutter und in
- Fig. 6: die Anordnung einer Schraube, die in Richtung der Längsachse zweier Platten geneigt angeordnet ist.

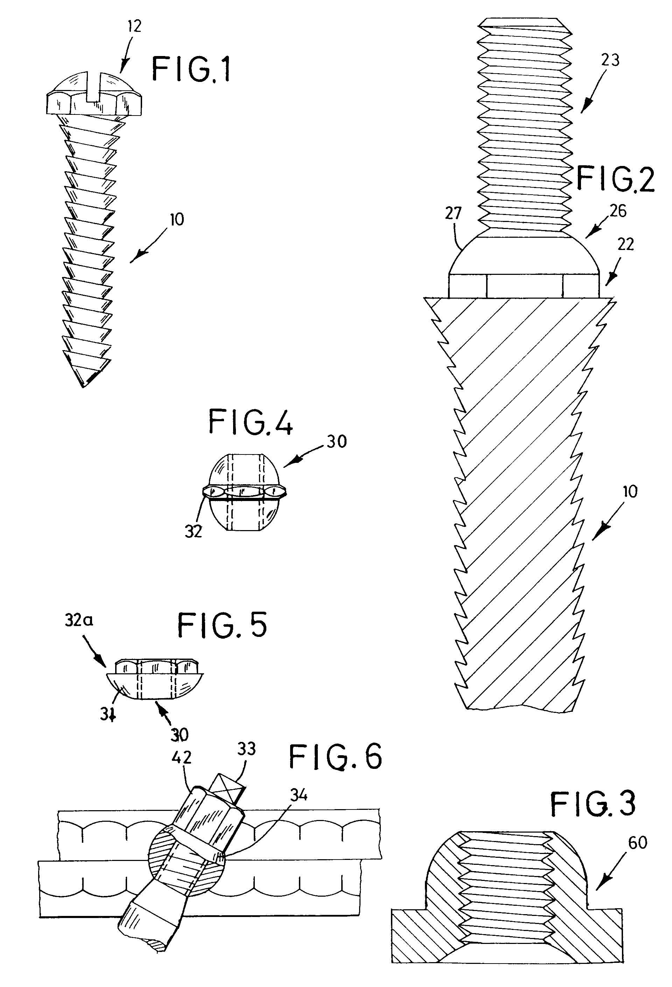

In Fig. 1 ist eine Schraube dargestellt, die einen Knochengewindebereich 10 und eine Handhabe 12 aufweist, wobei sich der Außendurchmesser des Knochengewindebereiches 10 zur Handhabe 12 hin erweitert und der Radiusanstieg auf einer Kurve erfolgt. Hierdurch wird die erforderliche Drehfestigkeit der Schraube in diesem Bereich erzielt.

In Fig. 2 ist eine Knochenschraube dargestellt, deren Knochengewindebereich 10 vom Normalbereich zur Handhabe 22 hin sich verdickt, wobei aber die Gewindetiefe gleichbleibt. Der Anstieg des Radius oder des Durchmessers erfolgt auf einer Kurve, d. h. der an sich übrige zylindrische Knochengewindebereich erweitert sich nach oben zur Handhabe 12 hin tulpenförmig. An den Knochengewindebereich 10 schließt sich eine Handhabe 22 an, die bei dem dargestellten Ausführungsbeispiel als Sechskantplatte ausgebildet ist. Eine solche Sechskantplatte schafft einen ebenen Oberflächenbereich, der das Ansetzen eines Betätigungswerkzeuges ermöglicht, daß die Platte gabelförmig umfaßt und damit nur an einer Seite offen ist und die Handhabe 22 an drei Seiten umgreift. An die plattenförmige Handhabe 22 schließt sich ein zylindrischer Bereich 26 an, der der Verstärkung und der Auflage eines Osteosynthesehilfsmittels, wie beispielsweise einer Platte oder eines Fixateurs dient. An diesen zylindrischen Bereich 26 schließt sich ein Maschinengewindebereich 23 an und der Übergang vom zylindrischen Bereich 26 zum Maschinengewindebereich 23 ist als sich konisch verjüngender Endbereich 27 ausgebildet. Wird dieser Endbereich 27 insbesondere konisch konvex ausgebildet, ist auch der Anschluß der Osteosyntheseplatte im Winkel zur Längsachse der Schraube möglich.

Die Fig. 4 bis 6 zeigen den Einsatz einer Mutter 30, die in Verbindung mit der in Fig. 2 dargestellten Schraube eingesetzt werden kann. Die in Fig. 4 dargestellte Mutter 30 weist zwei sphärische Bereiche und einen Handhabungsbereich 32 auf, während die in Fig. 5 dargestellte Mutter nur einen sphärischen Bereich 31 aufweist und der Handhabungsbereich 32a gegenüber dem sphärischen Bereich zurückversetzt ist. Auf dem sphärischen Bereich dieser Muttern 30 können sich entsprechende Osteosyntheseeinrichtungen, wie beispielsweise Osteosyntheseplatten, abstützen. Hierbei zeigt die Figur, daß durch die Ausbildung der Muttern 30 ein schräges Einsetzen der Knochenschrauben möglich ist, wobei in Fig. 6 eine Stützkugel 34 auf den Maschinengewindebereich 23 einer Knochenschraube aufgeschraubt ist. Diese Stützkugel 34 kann durch eine Kontermutter 42 festgelegt werden. Hier ist das obere Ende 33 der Schraube polygonal ausgebildet.

Schließlich zeigt Fig. 3 eine der Verstärkung dienende Hülse 60, die eine untere sphärische Ausnehmung und eine obere sphärische Wölbung besitzt. Diese Hülse 60 kann auf den Maschinengewindebereich 23 der Schraube aufgeschraubt werden.

## Patentansprüche

1. Schraube als Osteosynthesehilfsmittel für die Fixation von Knochen bzw. Knochenfragmenten mit einem Knochengewindebereich und einer Handhabe, wobei der Knochengewindebereich (10) unterhalb der Handhabe (12, 22) verstärkt ausgebildet ist, dadurch gekennzeichnet, daß sich der Außendurchmesser des Knochengewindebereiches (10) vom Normalbereich zur Handhabe (12) hin erweitert und der Radiusanstieg auf einer Kurve erfolgt (Fig. 1).

2. Schraube nach Anspruch 1, dadurch gekennzeichnet, daß der Außendurchmesser der Handhabe (22) kleiner als der maximale Außendurchmesser im Verstärkungsbereich ist (Fig. 2).

3. Schraube nach Anspruch 1 oder 2, dadurch gekennzeichnet, daß die Gewindetiefe des Knochengewindes (10) über die Länge der Schraube gesehen gleichbleibt.

4. Schraube nach einem der vorhergehenden Ansprüche, gekennzeichnet durch eine sich an die Handhabe (22) anschließende Verlängerung, die mit Maschinengewinde (23) ausgerüstet ist (Fig. 2).

5. Schraube nach einem der vorhergehenden Ansprüche, dadurch gekennzeichnet, daß sich an die Handhabe (22) in Richtung des Maschinengewindebereiches (23) ein gewindeloser Bereich (26) anschließt, dessen oberer Endbereich (27) als Auflage für eine Osteosyntheseplatte dienen kann und damit im Durchmesser größer ist als der Durchmesser des Maschinengewindebereiches, wobei gleichzeitig der gewindelose Bereich (26) eine Verstärkung oberhalb der Handhabe (22) schafft (Fig. 2).

6. Schraube nach Anspruch 5, dadurch gekennzeichnet, daß der Endbereich (27) konisch oder konvex oder sphärisch ausgebildet ist.

7. Schraube nach einem der vorhergehenden Ansprüche, dadurch gekennzeichnet, daß die Verstärkung durch eine mit dem Übergang zwischen Maschinengewindebereich (23) und Handhabe (22) zusammenwirkende und diesen Bereich stabilisierende Hülse (60) erfolgt (Fig. 3).

8. Schraube nach einem der vorhergehenden Ansprüche, dadurch gekennzeichnet, daß die Hülse (60) ein mit dem Maschinengewinde (23) zusammenwirkendes Innengewinde aufweist.

9. Schraube nach einem der vorhergehenden Ansprüche, gekennzeichnet durch eine mit dem Maschinengewindebereich (23) zusammenwirkende Mutter (30), die wenigstens eine sphärisch ausgebildete Anlagefläche (31) aufweist (Fig. 5).

10. Schraube nach Anspruch 8, dadurch gekennzeichnet, daß die Mutter (30) zwei sphärisch ausgebildete Oberflächenbereiche besitzt (Fig. 4).

11. Schraube nach Anspruch 8 oder 9, dadurch gekennzeichnet, daß die Mutter (30) mit einer Handhabe (32) ausgerüstet ist.

12. Schraube nach einem der vorhergehenden Ansprüche, dadurch gekennzeichnet, daß das Ende (33) des Maschinengewindebereiches (23) polygonal ausgebildet ist (Fig. 6).

13. Schraube nach einem der vorhergehenden Ansprüche, gekennzeichnet durch eine auf dem Maschinengewindebereich (23) aufschraubbare Stützkugel (34) (Fig. 6).

## Claims

1. A screw as auxiliary osteosynthetic agent for fixing bones or bone fragments, with a bone thread area and a handle, the bone thread area (10) being constructed reinforcedly below the handle (12, 22), characterized in that the external diameter of the bone thread area (10) widens from the normal area towards the handle (12) and the radius leading edge effects a curve (Fig. 1).

2. A screw according to claim 1, characterized in that the external diameter of the handle (22) is smaller than the maximum external diameter in the area of reinforcement (Fig. 2).

3. A screw according to claim 1 or claim 2, characterized in that the thread depth of the bone thread (10) remains the same when viewed over the length of the screw.

4. A screw according to any one of the preceding claims, characterized by an extension adjoining the handle (22), which extension is provided with a machine thread (23) (Fig. 2).

5. A screw according to any one of the preceding claims, characterized in that a thread-less area (26) adjoins the handle (22) in the direction of the machine thread area (23), the upper end area (27) of which thread-less area (26) may serve as a support for an osteosynthetic plate and thus has a larger diameter than the diameter of the machine thread area, the thread-less area (26) at the same time providing a reinforcement above the handle (22) (Fig. 2).

6. A screw according to claim 5, characterized in that the end area (27) is of conical or convex or spherical construction.

7. A screw according to any one of the preceding claims, characterized in that the reinforcement is effected by a sleeve (60) interacting with the transition between machine thread area (23) and handle (22) and stabilising this area (Fig. 3).

8. A screw according to any one of the preceding claims, characterized in that the sleeve (60) comprises an internal thread interacting with the machine thread (23).

9. A screw according to any one of the preceding claims, characterized by a nut (30) interacting with the machine thread area (23), which nut (30) comprises at least one spherically constructed bearing face (31) (Fig. 5).

10. A screw according to claim 8, characterized in that the nut (30) has two spherically constructed surface areas (Fig. 4).

11. A screw according to claim 8 or claim 9, characterized in that the nut (30) is provided with a handle (32).

12. A screw according to any one of the preceding claims, characterized in that the end (33) of the machine thread area (23) is of polygonal construction (Fig. 6).

13. A screw according to any one of the preceding claims, characterized by a supporting sphere (34) screwable on the machine thread area (23) (Fig. 6).

## Revendications

1. Vis auxiliaire d'ostéosynthèse pour la fixation d'os ou de fragments d'os comprenant une portion de filet à os et une prise, dans laquelle la portion de filet à os (10) est renforcée en-dessous de la prise (12, 22), caractérisée en ce que le diamètre extérieur de la portion de filet à os (10) s'élargit depuis la portion normale jusqu'à la prise (12) et l'augmentation de rayon se fait selon une courbe (Figure 1).

2. Vis selon la revendication 1, caractérisée en ce que le diamètre extérieur de la prise (22) est plus petit que le diamètre extérieur maximal dans la portion de renfort (Figure 2).

3. Vis selon la revendication 1 ou 2, caractérisée en ce que la profondeur du filet à os (10) reste la même sur la longueur de la vis.

4. Vis selon l'une ou l'ensemble des revendications précédentes, caractérisée en ce qu'elle comporte un prolongement se raccordant à la prise (22), qui est pourvu d'un filet machine (23) (Figure 2).

5. Vis selon l'une ou l'ensemble des revendications précédentes, caractérisée en ce qu'une portion sans filetage (26), dont la partie d'extrémité supérieure (27) peut servir d'appui à une plaque d'ostéosynthèse et a donc un plus grand diamètre que la portion de filet machine, se raccorde à la prise (22) en direction de la portion de filet machine (23), la portion sans filet (26) créant en même temps un renfort au-dessus de la prise (22) (Figure 2).

6. Vis selon la revendication 5, caractérisée en ce que la portion d'extrémité (27) est de forme conique, convexe ou sphérique.

7. Vis selon l'une ou l'ensemble des revendications précédentes, caractérisée en ce que le renfort est réalisé par un manchon (60) coopérant avec la transition entre la portion de filet machine (23) et la prise (22) et stabilisant cette portion (Figure 3).

8. Vis selon l'une ou l'ensemble des revendications précédentes, caractérisée en ce que le manchon (60) présente un filet intérieur coopérant avec le filet machine (23).

9. Vis selon l'une ou l'ensemble des revendications précédentes, caractérisée en ce qu'elle comporte un écrou (30) coopérant avec la portion de filet machine (23), qui présente au moins une surface d'appui (31) de forme sphérique (Figure 5).

10. Vis selon la revendication 8, caractérisée en ce que l'écrou (30) possède deux portions de surface de forme sphérique (Figure 4).

11. Vis selon la revendication 8 ou 9, caractérisée en ce que l'écrou (30) est pourvu d'une prise (32).

12. Vis selon l'une ou l'ensemble des revendications précédentes, caractérisée en ce que l'extrémité (33) de la portion de filet machine (23) est de forme polygonale (Figure 6).

13. Vis selon l'une ou l'ensemble des revendications précédentes, caractérisée en ce qu'elle comporte une bille d'appui (34) pouvant être vissée sur la portion du filet machine (23) (Figure 6).
